# EUROPEAN PATENT SPECIFICATION

(11) **EP 0 874 580 B1**
(45) Date of publication and mention of the grant of the patent: **03.03.2004**
(21) Application number: 96940643.8
(22) Date of filing: 04.12.1996
(51) Int. Cl.: A47L 15/00, A61G 9/02

(54) **A METHOD FOR STEAM DISINFECTION OF THE WASHING TANK OF AN AUTOMATIC DISHWASHER AND AN AUTOMATIC DISHWASHER FOR UTILIZING THE METHOD**
VERFAHREN ZUM DESINFIZIEREN DES SPÜLRAUMES EINER AUTOMATISCHEN GESCHIRRSPÜLMASCHINE UND AUTOMATISCHE GESCHIRRSPÜLMASCHINE ZUR DURCHFÜHRUNG DES VERFAHRENS
PROCEDE DE DESINFECTION A LA VAPEUR DE LA CUVE DE LAVAGE D'UN LAVE-VAISSELLE AUTOMATIQUE ET LAVE-VAISSELLE AUTOMATIQUE FAISANT INTERVENIR CE PROCEDE

(30) Priority: 05.12.1995 DK 137695
(43) Date of publication of application: 04.11.1998
(73) Proprietor: Ken Maskinfabrik A/S, 5672 Broby (DK)
(72) Inventor: KRISTENSEN, Hans, Sonderby, DK-5492 Vissenbjerg (DK); NIELSEN, Ulrich, Hein, Boe, DK-5690 Tommerup (DK)
(74) Representative: Holme, Edvard
(86) International application number: PCT/DK1996/000508
(87) International publication number: WO 1997/020493

(56) References cited:
- EP-A- 0 093 846
- DE-A- 2 900 957
- DE-A- 4 034 380
- FR-A- 1 565 407
- US-A- 4 135 531
- US-A- 4 235 642
- US-A- 4 279 384

## Description

The invention concerns a dishwasher and a process carried out by means of the dishwasher for, in institutions, where the hygienic requirements are high due to the danger of infection, washing surgical equipment, laboratory equipment, clinical equipment, bedpans etc..

The dishwasher is comprising a warm water tank connectable to a supply of fresh water, a heating element situated in the warm water tank for heating up water supplied to the warm water tank to the preferred washing temperature and for generating of steam, a washing tank for washing equipment placed in said washing tank, a number of flushing nozzles arranged in the washing tank, and pipe connections extending from the warm water tank to the flushing nozzles in the washing tank.

For hygienic considerations there is generally basic requirements for the arrangement of such dishwashers such as an independent warm water tank.

Water and/or steam in the dishwater usually will be contaminated with impurities and bacteria from the equipment to be washed. In some cases the pressure in the fresh water supply can, however, be lesser than in the dishwater, whereby the contaminated water in this can be sucked back into the fresh water supply. In order to prevent contamination of the fresh water in the water supply (warm and/or cold) this may therefore only be connected to the warm water tank via an air gap, since the contaminated water and/or steam cannot pass through this from the warm water tank to the fresh water supply.

Furthermore, the washing tank of these washing machines is disinfected with steam and thereby also the objects staying in the machine after the end of the washing process. The steam is supplied either from an external steam source or from an independently built-in steam source in the dishwasher.

Such dishwashers are also provided with a flushing pump for providing the necessary flushing pressure for the washing process, since the pressure from the fresh water supply cannot be utilised because of the previously mentioned air gap.

An example of a such dishwasher is indicated in EP 0 093 846 B1, where the dishwasher comprises a warm water tank, the supply pipe of which having an air gap for preventing contaminated water in the warm water tank to be sucked back into the water supply. The warm water tank does not have an internal heating element but is supplied with warm and cold water from external sources. After the termination of the washing process the dishwasher performs a disinfection of the washing tank by means of steam which is either produced in an independent steam generating unit somewhere else in the machine or is supplied through pipe connections from an external source of steam.

A dishwasher with internal warm water tank and an independent steam generating unit is known from EP 0 055 926 B1. The indicated dishwasher does not perform disinfection of the warm water tank and the pipe connections between this and the washing tank, which makes these washing machines unsuitable for performing a subsequent chilling flush necessary for the objects in the washing machine to be handled manually immediately after the end of the washing process. The reason for this is explained in detail below.

It has appeared that formation of bacteria often occurs inside the warm water tank and in the pipe system between this and the washing tank of the washing machine, since the temperature range within which the warm water tank operates at the washing process (40°C - 65°C) makes it possible for e.g. legionella bacteria to survive, since these are only killed at a higher temperature. It is true that the bacteria in the washing tank are killed at the steam disinfection, but at the performing of a subsequent chilling flush there may be supplied bacteriae from the warm water tank to the washing tank with the chilling flush water and thereby to the items washed in the machine when using the above mentioned dishwasher.

The solution to the bacteria problem in the warm water tank may consist in either omitting the chilling flush, which will lower the capacity of the machine, since the washed objects have to be cooled down by the air before they can be handled manually. Alternatively the working temperature of the warm water tank and thereby of the washing process may be elevated in general, so that the temperature of the warm water tank exceeds the survival temperature of the above mentioned type of bacteria. Thus it will be without any risk to perform a chilling flush after termination of the steam disinfection, but a general elevation of the working temperature will result in a considerable increase in the energy consumption of the washing process, which renders this solution unwanted.

Another solution could be that after performing the washing process the warm water tank is disinfected by supplying steam from a special internal or external steam source before it is filled up again, whereafter the chilling flush is performed with clean water supplied to the washing tank via the warm water tank. This procedure will be able to solve the problem, but should the occasion arise the establishment of a special steam producing unit is required, combined with control system, valves etc. for the washing machine, which hereby is made more expensive.

In FR 1.565.407 there is disclosed a system for improving a dishwasher for hospital equipment, instruments etc, and comprising a warm water tank with internal heating element, where the warm water tank is connected directly to the fresh water supply, so that the supply pressure is used as a flush water pressure. The heating element produces steam for disinfection the washing tank and the warm water tank together with the pipe connections in-between. However, a such device is outdated, viz. the previously mentioned hygienic requirements, and furthermore today there is required a greater flush water pressure than possible with a normal fresh water supply.

The invention provides a dishwasher of the kind mentioned in the opening paragraph according to claim 1.

The process according to claim 8 is carried out by means of the dishwasher of claim 1.

The air gap prevents that contaminated water and/or steam can be sucked back into the fresh water supply contaminating the fresh water. Therefore the pressure from the fresh water supply can not be used to drive the heated water from the warm water tank to the flushing nozzles in the washing tank. This part of the process is instead carried out by means of the flush water pump.

After termination of the washing process steam is in a simple way generated by using the same heating element. The steam is used for disinfection the warm water tank, the washing tank and the pipe connections there between. Chilling flushing therefore is performed from the newly disinfected tanks and pipe connections, whereby bacterial growth advantageously is hindered.

According to the invention the intake pipe between the flush water pump and the warm water tank can by an advantageous embodiment be arranged with its upper edge at a line defining a minimum water level, at which there is enough water left in the well for generating the quantity of steam required for disinfection the warm water tank and the washing tank and the pipe connections between the two tanks.

The warm water tank such has two functions, namely a function as a warm water tank with a heating function until the water level in the warm water tank has reached the upper edge of the intake pipe and thereafter a function as a steam generator for the production of steam for disinfection the warm water tank and washing tank before a chilling flush is performed.

For securing also disinfection of the flush pipe connections between the warm water tank and the washing tank the steam pipes can be connected with the flush pipe of the washing machine through pipe connections with check valves.

The check valves prevent the supply of flush water to the steam pipe system during the washing process. Furthermore, the warm water tank with the integrated steam generator will be much less costly to produce since there is only need for one tank comprising one heating element with attached electric installations as compared to two before.

In a specially preferred embodiment the flush pipe system is provided with a draining device for draining the pump housing and the flush pipe connections after finishing the washing, so that pipe connections as well as pump housing are open for the passage of steam. Hereby there is also achieved the advantage that also the pump housing is disinfected in connection with the steam disinfection before a possible chilling flushing.

For ensuring a sufficient steam flow through the pump housing the steam pipe in the top of the warm water tank may be provided with a controlable shut-off valve, so that the steam flow is guided through the pump housing and the flush pipes by closing the steam pipe.

The warm water tank with integrated steam generator according to the invention may further be utilised as an independent unit in connection with other disinfection washing machines.

The invention is described in more detail below with reference to the drawing, where
Fig. 1 shows a schematic drawing of a dishwasher with warm water tank and integrated steam generator according to the invention,
Fig. 2 shows the same as fig. 1, but where the flush water system i provided with a draining device, and

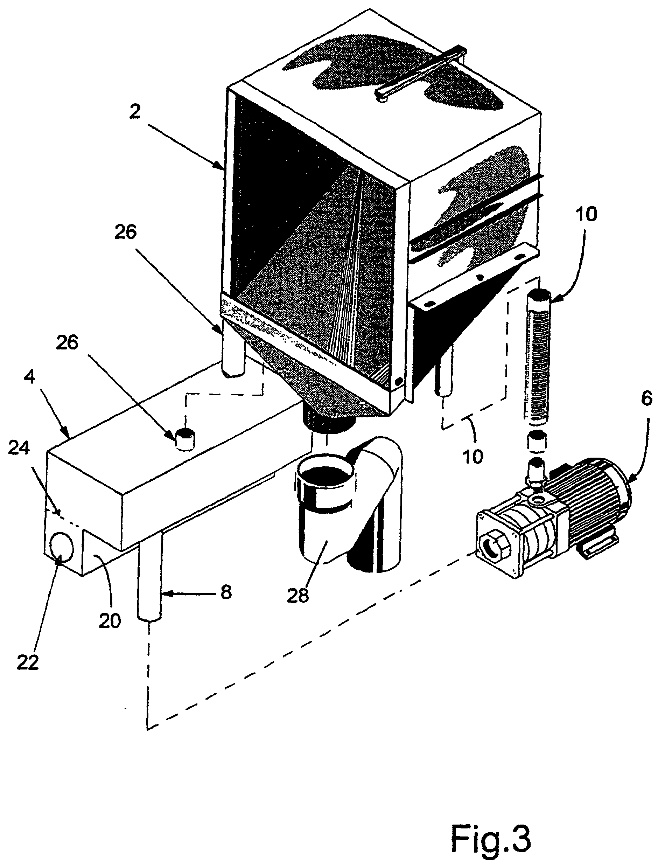

Fig. 3 is a perspective view of an embodiment of a dishwasher according to claim 1.

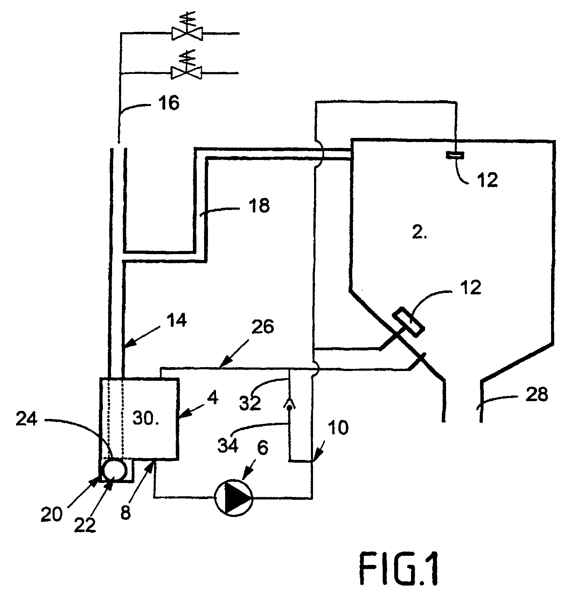

Fig. 1 shows a dishwasher comprising a washing tank 2, a warm water tank 4, and a flush water pump 6 with an intake connected with the tank through a pipe connection 8. The pump 6 is connected to flushing nozzles 12 in the washing tank 2 through flush pipe 10. The warm water tank 4 is supplied with fresh water through a pipe 14, which by a not shown air gap is connected to a supply 16 of warm and cold water. The pipe 14 has a branch 18 connected to the washing tank 2 for ventilating the latter.

The peculiar feature of the warm water tank 4 is that it comprises a bottom well 20 in which the pipe 14 is led down to a level close to its bottom. In the well 20 there is also provided a heating element 22 for heating up fresh water supplied to the tank to the preferred washing temperature, and for the generation of steam.

The intake pipe 8 of the pump is connected in such a way to the warm water tank 4 that it only can be emptied to the level at the line 24, which means that the volume of the tank down to the level at the line 24 is adjusted to the water volume used at the whole washing process in the dishwasher, since the flushing water is not recirculated in the washing process in this type of dishwashers.

A pipe 26 is connected to the top of the tank for conducting steam to the washing tank 2 for disinfection the latter.

The function of the warm water tank is that the tank 4 is filled with fresh water from the supply 16 for warm and cold water through the pipe 14. The heating element 22 is activated, if necessary, whereby the water is heated to the desired temperature. The heated water is supplied to the flushing nozzles 12 in the washing tank 2 of the dishwasher by the pump 6, the pipe 8, and the flush pipe connection 10, and the flush water is discharged directly to the drain 28 of the dishwasher.

At the finish of the washing process the water level in the tank 4 is lowered to the level 24, which is defined by the upper edge of the pipe 8. Steam disinfection of the washing tank 2 of the dishwasher hereby takes place by heating element 22 heating a minimum quantity of water, i.e. the portion of water left in the well 20, so that steam is generated and spread in the tank space 30 and from this to the washing tank 2 of the dishwasher through the pipe connection 26 in the top of the warm water tank 4, whereby the latter and the washing tank 2 is disinfected with steam.

No water is supplied to the warm water tank during the disinfection process, since the upper edge of the intake to the feed pipe 8 for the flush water pump 6 defines a certain minimum water level 24 at which there is enough water left in the well 20 for the steam production required for the disinfection process.

By the extending of the pipe 14 to a level close to the bottom of the well 20 there is created a trap preventing steam to escape through the pipe 14.

By the steam disinfection process there is performed disinfection of the washing tank 2 as well as the warm water tank 4 by using the warm water tank according to the invention, whereby possible bacteria in it are killed. Fresh water is filled into the warm water tank after ending disinfection of the washing tank and the warm water tank.

The water is heated partly by the residual heat in the tank walls and partly by means of the heating element 22. In certain cases there may be performed a chilling flush in the dishwasher, which now may be performed with completely germ-free water from the disinfected warm water tank in accordance with the Swedish recommendation for hygiene, Spri no. 742 01.

As appearing from Fig. 1 the steam pipe 26 may be provided with a branch 32 furnished with a check valve 34, so that the greater part of the flush pipes 10 and the flush nozzles 12 are supplied with steam and are thereby also disinfected.

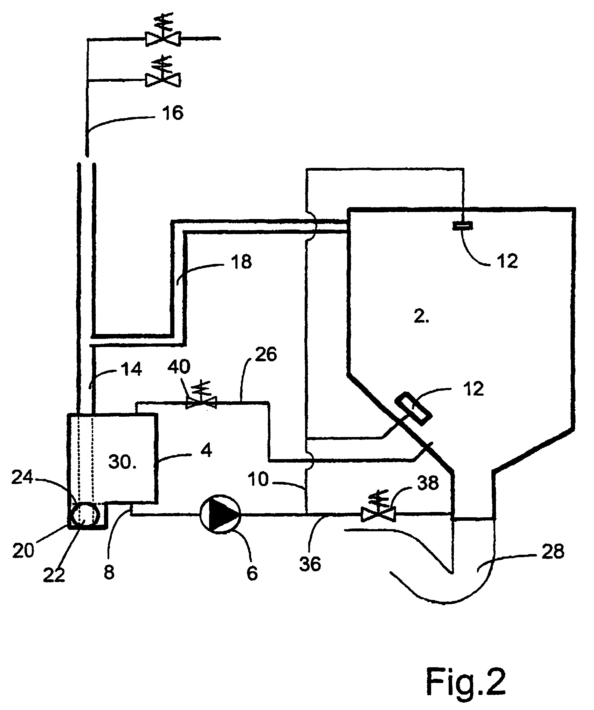

In a specially preferred embodiment shown in Fig. 2 the flush pipe 10 is provided at the lowest point with a drain pipe 36 connected to the drain 28 from the washing tank 2.

The drain pipe 36 is provided with an electromagnetic valve 38, the activation of which results in opening the pipe connection 36, so that the pump 6 and pipe connections 8,10 are emptied into the drain 28. Thereby the pipe connections and the pump housing are open for the passage of steam from the tank space 30 of the warm water tank, which results in that by this embodiment of the washing machine according to the invention there is achieved a complete steam disinfection of both warm water tank 4, washing tank 2, draining pipe 8, flush water pump 6, flush pipe 10, and flushing nozzles 12 in the washing tank.

For ensuring a flow of steam through the pipe connections 8,10 and the flushing nozzles 12, there may be provided an electromagnetic valve 40 (Fig. 2) on the steam pipe connection 26, which temporarily closes the steam flow through the steam pipe 26, which by continuing steam generation in the tank 4 will force the steam flow through the flush water pump 6 and the pipe connections 8,10 together with the flushing nozzles 12. The electromagnetic valve 40 is coupled to the remaining control system of the washing machine for controlling the washing and disinfection process.

In a further, not shown embodiment, the warm water tank may be furnished with level sensors and a control system so that during the steam generation there is supplied water to the well 20 through the pipe 14 in order to maintain the water level designated by the line 24.

By the dishwasher according to the invention it is implied that the dishwasher may comprise essential automatic control system for controlling and regulating the washing process as well as the steam disinfection, and also that the dishwasher naturally comprises means for the dosing of additives required for the washing and disinfection process.

## Claims

1. A dishwasher for washing surgical equipment, laboratory equipment, clinical equipment, bedpans etc., said dishwasher comprising
- a warm water tank (4) connectable to a supply of fresh water (16),
- a heating element (22) situated in the warm water tank (4) for heating up fresh water supplied to the warm water tank (4) to the preferred washing temperature and for generating steam,
- a washing tank (2) provided with a drain (28),
- flushing nozzles (12) arranged in the washing tank (2), and
- pipe connections (8,10) extending from the warm water tank (4) to the flushing nozzles (12) in the washing tank (2),
**characterized in,**
- **that** between the warm water tank (4) and the supply of fresh water (16) an air gap is arranged,
- **that** the pipe connections (8,10) consist of an intake pipe (8) and a flush pipe (10),
- **that** a flush water pump (6) is connected with the warm water tank (4) through the intake pipe (8),
- **that** the flush water pump (6) is connected with the flushing nozzles (12) through the flush pipe (10),
- **that** a not emptyable and permanently wet well (20) is formed in the warm water tank (4),
- **that** the intake of the pipe (8) for the pump (6) has an upper edge that defines a minimum water level at which there is enough water left in the well (20) for the steam production required for the disinfection process,
- **that** the heating element (22) is situated in said wet well (20), and
- **that** a steam pipe (26) is extending from the top of the warm water tank (4) to the washing tank (2).

2. A dishwasher according to claim 1, **characterized in that** the intake pipe (8) is connected to the warm water tank (4) with its upper edge at a line (24) defining a minimum water level, at which there is enough water left in the well (20) for generating the quantity of steam required for disinfecting the warm water tank (4) and the washing tank (2).

3. A dishwasher according to claim 2, **characterized in that** the intake pipe (8) is connected to the warm water tank (4) with its upper edge at a line (24) defining a minimum water level, at which there is enough water left in the well (20) for generating the quantity of steam required for further disinfecting the pipe connections (8,10), the pump (6) and the flushing nozzles (12).

4. A dishwasher according to any of the claim 1, 2 or 3, **characterized in that** the warm water tank (4) is connectable to the supply of fresh water (16) via a supply pipe (14) which is extending down into the well (20) close to the bottom of this.

5. A dishwasher according to any of the claims 1 - 4, **characterized in that** the steam pipe (26) is connected to the flush pipe (10) via a branch (32) furnished with a check valve (34).

6. A dishwasher according to any of the claims 1 - 5, **characterized in that** the flush pipe (10) is connected at its lowest point to a drain pipe (36) connected to a drain (28) from the washing tank (2), and that an electromagnetic valve (38) is inserted in said drain pipe (36).

7. A dishwasher according to any of the claims 1 - 6, **characterized in that** an electromagnetic control valve (40) is inserted in the steam pipe (26).

8. A process carried out by means of the dishwasher according to the claims 1 - 7 for washing surgical equipment, laboratory equipment, clinical equipment, bedpans etc., comprising
- filling fresh water into the warm water tank (4) via the air gap,
- heating the water in the warm water tank (4) to the desired washing temperature,
- supplying the water heated in the warm water tank (4) via the intake pipe (8), pump (6) and flush pipe (10) to the flushing nozzles (12) in the washing tank (2),
- discharging the flush water to the drain (28) of the washing tank (2)
- further heating a minimum quantity of water in the warm water tank (4) to such a high temperature that steam is generated in the tank space (30) of the warm water tank (4), said minimum quantity of water being defined by the upper edge of the intake to the pipe (8) for feeding the pump (6) , and
- conducting steam generated in the tank space (30) of the warm water tank (4) from said tank space (30) to the washing tank (2) for disinfecting said washing tank (2).

9. A process according to claim 8, **characterized in that** steam is spread to the washing tank (2) from the top of the warm water tank (4).

10. A process according to claim 8 or 9, **characterized in that** steam furthermore is supplied to a greater part of the flush pipe (10) and the flushing nozzles (12) for disinfecting said flush pipe (10) and said flushing nozzles (12).

11. A process according to claim 8, 9 or 10, **characterized in that** the process further comprises emptying the pump (6) and the pipe connections (8,10) into the drain (28) thereby opening the housing of the pump (6) and the pipe connections (8,10) for passage of the steam from the tank space (30).

12. A process according to any of the claims 8 - 11, **characterized in** pumping water heated in the warm water tank (4) to the flushing nozzles (12) in the washing tank (2) until a minimum water level is achieved, at which there is portion of water left in the well (20) for generating the quantity of steam required for disinfecting the warm water tank (4) and the washing tank (2) and the pipe connections between the two tanks (4;2).

13. A process according to any of the claims 8 - 12, **characterized in** using the same heating element (22) for heating the washing water in the warm water tank (4) to the temperature desired for washing the equipment in the washing tank (2) and afterwards generating steam from the water left in the well (20).

## Patentansprüche

1. Spülmaschine zum Waschen von chirurgischer Gerätschaften, Laborgerätschaften, klinischen Gerätschaften, Bettpfannen etc. mit einem an eine Frischwasserversorgung (16) anschließbaren Warmwassertank (4), einem Heizelement (22), das sich in dem Warmwassertank (4) zur Erwärmung des für den Warmwassertank (4) bereitgestellten Frischwassers auf eine bevorzugte Temperatur und zur Erzeugung von Dampf befindet, einem Waschtank (2) mit einem Abfluss (28), im Waschtank (2) angeordneten Spüldüsen (12) und sich vom Warmwassertank (4) zu den Spüldüsen (12) im Waschtank (2) erstreckenden Rohrverbindungen (8, 10),
**dadurch gekennzeichnet, dass**
zwischen dem Warmwassertank (4) und der Frischwasserversorgung (16) ein Luftzwischenraum vorgesehen ist,
die Rohrverbindungen (8, 10) aus einem Zuflussrohr (8) und einem Spülrohr (10) bestehen,
eine Spülwasserpumpe (6) mit dem Warmwassertank (4) durch das Zuflussrohr (8) verbunden ist,
die Spülwasserpumpe (6) durch das Spülrohr (10) mit den Spüldüsen (12) verbunden ist,
ein nicht entleerbarer und ständig nasser Schacht (20) im Warmwassertank (4) geformt ist,
der Zufluss des Rohrs (8) für die Pumpe (6) eine obere Kante besitzt, die einen minimalen Wasserstand definiert, bei dem genügend, für die Erzeugung von Dampf für den Desinfektionsvorgang notwendige Wasser in dem Schacht (20) vorhanden ist,
sich das Heizelement (22) im Schacht (20) befindet und
sich ein Dampfrohr (26) von der Oberseite des Warmwassertanks (4) zum Waschtank (2) erstreckt.

2. Spülmaschine nach Anspruch 1, **dadurch gekennzeichnet, dass** das Zuflussrohr (8) derart mit dem Warmwassertank (4) verbunden ist, dass seine obere Kante an einer Linie (24) verläuft, die den minimalen Wasserstand definiert, bei dem genügend Wasser im Schacht (20) vorhanden ist, um die Menge Dampf zu erzeugen, die für die Desinfektion des Warmwassertanks (4) und des Waschtanks (2) nötig ist.

3. Spülmaschine nach Anspruch 2, **dadurch gekennzeichnet, dass** das Zuflussrohr (8) derart mit dem Warmwassertank (4) verbunden ist, dass seine obere Kante an einer Linie (24) verläuft, die den minimalen Wasserstand definiert, bei dem genügend Wasser im Schacht (20) vorhanden ist, um die Menge Dampf zu erzeugen, um zusätzlich die Rohrverbindungen (8, 10), die Pumpe (6) und die Dampfdüsen (12) zu desinfizieren.

4. Spülmaschine nach einem der Ansprüche 1 - 3, **dadurch gekennzeichnet, dass** der Warmwassertank (4) an die Frischwasserversorgung (16) über eine Versorgungsleitung (14) angeschlossen werden kann, die sich nach unten bis nahe an den Boden des Schachts (20) erstreckt.

5. Spülmaschine nach einem der Ansprüche 1 - 4, **dadurch gekennzeichnet, dass** das Dampfrohr (26) an das Spülrohr (10) über eine Verzweigung (32) angeschlossen ist, die mit einem Kontrollventil (34) versehen ist.

6. Spülmaschine nach einem der Ansprüche 1 - 5, **dadurch gekennzeichnet, dass** das Spülrohr (10) an seinem niedrigsten Punkt an ein Abflussrohr (36), das mit dem Abfluss (28) des Waschtanks (2) verbunden ist, angeschlossen ist und dass ein elektromagnetisches Ventil (38) in dieses Abflussrohr (36) eingefügt ist.

7. Spülmaschine nach einem der Ansprüche 1 - 6, **dadurch gekennzeichnet, dass** ein elektromagnetisches Kontrollventil (40) in das Dampfrohr (26) eingefügt ist.

8. Ein Verfahren, das mittels einer Spülmaschine nach einem der Ansprüche 1 - 6 zum Waschen von chirurgischer Gerätschaften, Laborgerätschaften, klinischer Gerätschaften, Bettpfannen etc. ausgeführt wird,
**gekennzeichnet durch**
Füllen des Frischwassers in den Warmwassertank (4) über den Luftzwischenraum,
Erhitzen des Wassers im Warmwassertank (4) auf die gewünschte Waschtemperatur,
Versorgen der Spüldüsen (12) im Waschtank (2) mit dem im Warmwassertank (4) erwärmten Wasser über das Zuflussrohr (8), die Zuflusspumpe (6) und das Zuflussspülrohr (10),
Entsorgen des Spülwassers über den Abfluss (28) des Waschtanks (2),
weiteres Erhitzen einer minimalen Menge Wassers im Warmwassertank (4) auf eine derart hohe Temperatur, dass im Tankraum (30) des Warmwassertanks (4) Dampf erzeugt wird, wobei die minimale Menge des Wassers **durch** die obere Kante des Zuflusses des Rohrs (8) zur Versorgung der Pumpe (6) begrenzt ist und
Leiten des im Tankraum (30) des Warmwassertanks (4) erzeugten Dampfes vom Tankraum (30) zum Waschtank (2) zur Desinfektion des Waschtanks (2).

9. Verfahren nach Anspruch 8, **dadurch gekennzeichnet, dass** der Dampf von der Oberseite des Warmwassertanks (4) in den Waschtank (2) verbreitet wird.

10. Verfahren nach Anspruch 8 oder 9, **dadurch gekennzeichnet, dass** der Dampf weiterhin an einen größeren Teil des Spülrohrs (10) und die Spüldüsen (12) zur Desinfektion des Spülrohrs (10) und der Spüldüsen (12) geführt wird.

11. Verfahren nach Anspruch 8, 9 oder 10, **dadurch gekennzeichnet, dass** das Verfahren weiterhin das Entleeren der Pumpe (6) und der Rohrverbindungen (8, 10) in den Abfluss (28) umfasst, wodurch das Gehäuse der Pumpe (6) und die Rohrverbindungen (8, 10) zur Passage des Dampfes vom Tankraum (30) geöffnet werden.

12. Verfahren nach einem der Ansprüche 8 bis 11, **dadurch gekennzeichnet, dass** das im Warmwassertank (4) erhitzte Wasser zu den Spüldüsen (12) im Waschtank (2) gepumpt wird bis ein minimaler Wasserstand erreicht wird, bei dem ein Teil des Wassers im Schacht (20) verbleibt, um eine zur Desinfektion des Warmwassertanks (4) und des Waschtanks (2) und der Rohrverbindungen zwischen den beiden Tanks (4;2) benötigte Menge Dampf zu erzeugen.

13. Verfahren nach einem der Ansprüche 8 bis 12, **dadurch gekennzeichnet, dass** das selbe Heizelement (22) zur Erwärmung des Waschwassers im Warmwassertank (4) auf eine gewünschte Temperatur zum Waschen der Ausrüstung im Waschtank (2) anschließend zur Erzeugung von Dampf aus dem im Schacht (20) verbliebenem Wasser benutzt wird.

## Revendications

1. Un lave-vaisselle destiné à laver du matériel chirurgical, du matériel de laboratoire, du matériel de clinique, des bassins de lit, etc..., ledit lave-vaisselle comportant
- une cuve d'eau chaude (4) pouvant être raccordée à une alimentation en eau propre (16),
- un élément chauffant (22) se trouvant dans la cuve d'eau chaude (4) et servant à chauffer l'eau propre amenée à la cuve d'eau chaude (4) à la température de lavage choisie et à générer de la vapeur,
- une cuve de lavage (2) munie d'une voie d'écoulement (28),
- des buses de rinçage (12) disposées dans la cuve de lavage (2), et
- des raccords de tuyaux (8,10) s'étendant de la cuve d'eau chaude (4) jusqu'aux buses de rinçage (12) dans la cuve de lavage (2),
**caractérisé en ce**
- **qu'**un intervalle d'air est aménagé entre la cuve d'eau chaude (4) et l'alimentation en eau propre (16),
- **que** les raccords de tuyaux (8,10) se composent d'un tuyau d'entrée (8) et d'un tuyau de rinçage (10),
- **qu'**une pompe d'eau de rinçage (6) est raccordée à la cuve d'eau chaude (4) par le tuyau d'entrée (8),
- **que** la pompe d'eau de rinçage (6) est raccordée aux buses de rinçage (12) par le tuyau de rinçage (10),
- **qu'**un puits ne pouvant être vidé et toujours immergé (20) se forme dans la cuve d'eau chaude (4),
- **que** l'entrée du tuyau (8) destiné à la pompe (6) possède un bord supérieur définissant un niveau d'eau minimum auquel il reste assez d'eau dans le puits (20) pour produire la vapeur nécessaire au processus de désinfection,
- **que** l'élément chauffant (22) se trouve dans ledit puits immergé (20),et
- **qu'**un tuyau de vapeur (26) s'étend du haut de la cuve d'eau chaude (4) à la cuve de lavage (2).

2. Un lave-vaisselle selon la revendication 1, **caractérisé en ce que** le tuyau d'entrée (8) est raccordé à la cuve d'eau chaude (4), son bord supérieur se trouvant à hauteur d'une ligne (24) définissant un niveau d'eau minimum auquel il reste assez d'eau dans le puits (20) pour générer la quantité de vapeur nécessaire à la désinfection de la cuve d'eau chaude (4) et de la cuve de lavage (2).

3. Un lave-vaisselle selon la revendication 2, **caractérisé en ce que** le tuyau d'entrée (8) est raccordé à la cuve d'eau chaude (4), son bord supérieur se trouvant à hauteur d'une ligne (24) définissant un niveau d'eau minimum auquel il reste assez d'eau dans le puits (20) pour générer la quantité de vapeur nécessaire pour désinfecter en plus les raccords de tuyaux (8,10), la pompe (6) et les buses de rinçage (12).

4. Un lave-vaisselle selon l'une quelconque des revendications 1, 2 ou 3, **caractérisé en ce que** la cuve d'eau chaude (4) peut être raccordée à l'alimentation en eau propre (16) par l'intermédiaire d'un tuyau d'alimentation (14) se prolongeant vers le bas jusque dans le puits (20), près du fond de celui-ci.

5. Un lave-vaisselle selon l'une quelconque des revendications 1 à 4, **caractérisé en ce que** le tuyau de vapeur (26) est raccordé au tuyau de rinçage (10) par l'intermédiaire d'un embranchement (32) muni d'un clapet de non-retour (34).

6. Un lave-vaisselle selon l'une quelconque des revendications 1 à 5, **caractérisé en ce que** le tuyau de rinçage (10) est raccordé, à son point le plus bas, à un tuyau d'écoulement (36) raccordé à une voie d'écoulement (28) à partir de la cuve de lavage (2), et **en ce qu'**une vanne électromagnétique (38) est insérée dans ledit tuyau d'écoulement (36).

7. Un lave-vaisselle selon l'une quelconque des revendications 1 à 6, **caractérisé en ce qu'**une vanne électromagnétique de commande (40) est insérée dans le tuyau de vapeur (26).

8. Un procédé, réalisé à l'aide du lave-vaisselle selon les revendications 1 à 7, destiné au lavage de matériel chirurgical, de matériel de laboratoire, de matériel clinique, de bassins de lit, etc... consistant à
- remplir la cuve d'eau chaude (4) avec de l'eau propre via l'intervalle d'air,
- chauffer l'eau dans la cuve d'eau chaude (4) à la température de lavage désirée,
- amener l'eau chauffée dans la cuve d'eau chaude (4), via le tuyau d'entrée (8), la pompe (6) et le tuyau de rinçage (10) vers les buses de rinçage (12) dans la cuve de lavage (2),
- évacuer l'eau de rinçage vers la voie d'écoulement (28) de la cuve de lavage (2),
- chauffer ensuite une quantité minimum d'eau dans la cuve d'eau chaude (4) jusqu'à une température telle que de la vapeur est générée dans l'espace de réserve (30) de la cuve d'eau chaude (4), ladite quantité minimum d'eau étant définie par le bord supérieur de l'entrée du tuyau (8) destiné à alimenter la pompe (6), et
- conduire la vapeur générée dans l'espace de réserve (30) de la cuve d'eau chaude (4) depuis ledit espace de réserve (30) jusqu'à la cuve de lavage (2) afin de désinfecter ladite cuve de lavage (2).

9. Un procédé selon la revendication 8, **caractérisé en ce que** de la vapeur se répand jusqu'à la cuve de lavage (2) depuis le haut de la cuve d'eau chaude (4).

10. Un procédé selon la revendication 8 ou 9, **caractérisé en ce que** de la vapeur est en outre amenée jusqu'à une partie assez importante du tuyau de rinçage (10) et des buses de rinçage (12) afin de désinfecter ledit tuyau de rinçage (10) et lesdites buses de rinçage (12).

11. Un procédé selon la revendication 8, 9 ou 10, **caractérisé en ce que** le procédé consiste en outre à vider la pompe (6) et les raccords de tuyaux (8,10) dans la voie d'écoulement (28) en ouvrant pour cela le logement de la pompe (6) et les raccords de tuyaux (8,10) pour laisser passer la vapeur depuis l'espace de réserve (30).

12. Un procédé selon l'une quelconque des revendications 8 à 11, **caractérisé en ce que** de l'eau chauffée dans la cuve d'eau chaude (4) est pompée vers les buses de rinçage (12) dans la cuve de lavage (2) jusqu'à ce qu'on obtienne un niveau d'eau minimum permettant de laisser une partie de l'eau dans le puits (20) afin de générer la quantité de vapeur nécessaire à la désinfection de la cuve d'eau chaude (4) et de la cuve de lavage (2) et des raccords de tuyaux entre les deux cuves (4 ; 2).

13. Un procédé selon l'une quelconque des revendications 8 à 12 **caractérisé en ce qu'**on utilise le même élément chauffant (22) pour chauffer l'eau de lavage dans la cuve d'eau chaude (4) à la température souhaitée afin de laver le matériel dans la cuve de lavage (2) et pour ensuite générer de la vapeur à partir de l'eau restant dans le puits (20).
